(19) Europäisches Patentamt
European Patent Office
Office européen des brevets

(11) Publication number: **0 060 059**
**B1**

(12) # EUROPEAN PATENT SPECIFICATION

(45) Date of publication of patent specification: **21.08.85**

(51) Int. Cl.$^4$: **C 12 Q 1/46**

(21) Application number: **82300965.9**

(22) Date of filing: **25.02.82**

(54) **Method for determining the activity of cholinesterase and diagnostic solution for use therein.**

(30) Priority: **04.03.81 JP 29812/81**
**08.04.81 JP 51707/81**

(43) Date of publication of application:
**15.09.82 Bulletin 82/37**

(45) Publication of the grant of the patent:
**21.08.85 Bulletin 85/34**

(84) Designated Contracting States:
**CH DE FR GB IT LI NL**

(56) References cited:
**EP-A-0 067 234**
**GB-A-2 018 988**
**US-A-3 959 351**

**CHEMICAL ABSTRACTS, vol. 71, no. 1, 7th July 1969, page 289, no. 3153d, Columbus Ohio (USA).**

(73) Proprietor: **FUJIREBIO KABUSHIKI KAISHA also trading as FUJIREBIO INC.**
**6-7, Shimoochiai 4-chome Shinjuku-ku**
**Tokyo 161 (JP)**

(72) Inventor: **Kasahara, Yasushi**
**310, Estate Nagayama 3-4-4, Nagayama, 3-chome**
**Tama-shi Tokyo (JP)**
Inventor: **Ashihara, Yoshihiro**
**28, Harumicho 1-chome**
**Fuchu-shi Tokyo (JP)**
Inventor: **Sugiyama, Masami**
**2-9-19, Motoyokoyamamachi**
**Hachioji-shi Tokyo (JP)**
Inventor: **Harada, Takahiro**
**6-62, Ondacho 1-chome**
**Ube-shi Yamaguchi-ken (JP)**

(74) Representative: **Silverman, Warren et al**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description for the Contracting State IT**

This invention relates to a method for determining the activity of cholinesterase and to a diagnostic solution for use therein.

Two kinds of cholinesterase are known, these being generally referred to as true-cholinesterase and pseudocholinesterase. True-cholinesterase can be found in human red blood cells and nerve tissues. Pseudo-cholinesterase can be found in human sera and pancreas. Cholinesterase can decompose acylcholine (i.e. acetylcholine) to choline and acid (acetic acid).

When a person is subject to hepatic diseases or anaemia, the amount of cholinesterase in the blood decreases, and when a person is subject to nephrosis, diabetes mellitus or diseases of the nervous system, the amount of cholinesterase increases. A diagnosis of the diseases mentioned above can be made by measuring the level of cholinesterase in the blood. Therefore, the accurate determination of the activity of cholinesterase contained in a serum sample is significant from both the physiological and clinical viewpoints. The conventional methods for cholinesterase determination include (1) the Takahashi and Shibata method in which acetyl choline is used as a substrate and variation of the pH resulting from its decomposition is measured and (2) a method in which benzoylcholine is used as a substrate, choline liberated from the benzoylcholine is oxidized with cholinoxidase to liberate hydrogen peroxide ($H_2O_2$), the hydrogen peroxide is subjected to reaction with phenol and 4-aminoantipyrine in the presence of peroxidase to produce a quinonimine dye, and the concentration of the quinonimine dye produced is colorimetrically measured.

However, such conventional methods have disadvantages. The above method (1) requires complicated steps and the above method (2) does not give very precise results.

It is an object of the present invention to provide a simple method for determining the activity of cholinesterase.

According to the present invention, there is provided a method of determining the activity of cholinesterase comprising the steps of:
mixing a liquid containing a cholinesterase with a substrate solution containing hydroxybenzoate hydroxylase, nicotinamide adenine dinucleotide phosphate in reduced form (NADPH) and m- or p-hydroxybenzoyl choline having the following formula:

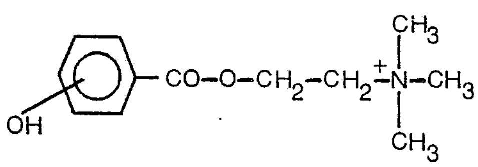

wherein the hydroxyl group is in the m- or p-position and
measuring the decrease in absorbance of light by NADPH as a measure of the activity of cholinesterase in the solution thereby indicating the consumption of NADPH in accordance with the amount of cholinesterase present in said liquid.

Hydroxybenzoyl choline having the following formula (with the OH group being m- or p-) is used as a synthetic substrate:

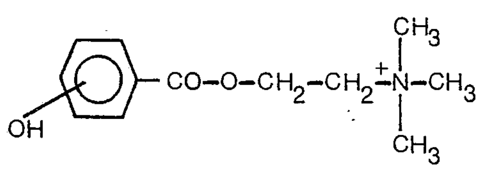

A cholinesterase-containing liquid such as a serum is added to a solution containing hydroxybenzoate hydroxylase, nicotinamide adenine dinucleotide phosphate in reduced form (hereinafter referred to as NADPH) and hydroxybenzoyl choline. Hydroxybenzoic acid is produced from hydroxybenzoyl choline by the action of cholinesterase. The hydroxybenzoic acid is converted to dihydroxybenzoic acid by the action of hydroxybenzoate hydroxylase in the presence of NADPH. The consumption of NADPH is measured by measuring the decrease in absorbance of light by NADPH. The cholinesterase activity of the serum is determined from the decrease in absorbance by calculation.

In the procedure mentioned above, one may use p-hydroxybenzoate hydroxylase, in which case p-hydroxybenzoyl choline is used as the substrate or one may use m-hydroxybenzoate hydroxylase, in which case the substate is m-hydroxybenzoyl choline.

The following examples illustrate the invention:

Example 1 (Preparative)

The synthetic substrate which is used in the method of the present invention, a hydroxybenzoyl choline, was synthesized as follows:

*(A) Synthesis of p-hydroxybenzoyl choline iodide:*

0.1 M of ethylene chlorohydrin and 0.1 M of p-hydroxybenzoic acid were added to 50 ml of chloroform, and to the mixture was added 0.5 ml of fluorosulphuric acid. After the mixture had been kept at 60°C for 4 days while under reflux, the mixture was heated at a temperature of 85°C on a water bath to remove the solvent. After the residue had been dissolved in chloroform and the solution had been washed three times with a 1% aqueous solution of $NaHCO_3$ and then three times with distilled water, the layer of chloroform was separated off and then chloroform was evaporated from the chloroform solution to leave a residue of 2-chloroethyl-p-hydroxybenzoate.

170 g (1 M) of anhydrous sodium iodide were then added to 1.2 l of methyl ethyl ketone and the mixture was heated on a steam bath for one hour. 0.9 M of 2-chloro-ethyl-p-hydroxybenzoate was added to the mixture and reaction was allowed to take place for 24 hours. After the precipitate which formed was filtered off the filtrate was washed with a 10% aqueous solution of sodium bisulphite and a 5% aqueous solution of sodium bicarbonate, and was then washed with 100 ml of distilled water.

The layer of methyl ethyl ketone which formed was separated off and 7 g of anhydrous magnesium sulphate was added to the methyl ethyl ketone layer to achieve drying of it. The solid matter was removed by filtration and the filtrate obtained was heated in an evaporator to remove the solvent and yield 2-iodoethyl-p-hydroxybenzoate.

0.7 M of the 2-iodoethyl-p-hydroxybenzoate thus formed was dissolved in 200 ml of acetone, and to the solution were added 270 ml of a 19.5% solution of trimethylamine in acetone. Reaction was then allowed to take place for 24 hours. The precipitate produced was filtered off, and the acetone layer was separated off and put in a vacuum desiccator to remove acetone. 20 g of p-hydroxybenzoyl choline iodide were obtained as a residue.

*(B) Synthesis of m-hydroxybenzoyl choline chloride:*

A mixture of 5 g of m-hydroxybenzoic acid, 5 g of choline chloride and 5 ml of 18N $H_2SO_4$ was heated under reflux for 26 hours and then the pH of the reaction mixture was adjusted to 4.0.

The solid matter present was filtered off, and the filtrate was concentrated under reduced pressure. The concentrate was separated by silica gel chromatography to give m-hydroxybenzoyl choline chloride.

Example 2

To determine the activity of cholinesterase which is contained in a serum, 5 ul of the serum were added to 1 ml of a solution (pH 7.5) containing 0.1 mM of p-hydroxybenzoyl choline iodide, 0.6 U of p-hydroxybenzoate hydroxylase, 0.2 mM of NADPH and 20 mM of phosphate buffer. The decrease of the absorbance by the solution was measured at 340 nm of light at a temperature of 30°C during the course of the reaction. During the determination, use was made of a reaction rate analyser (LKB 2086 made and sold by Clinicon Co., Sweden) in which the time lag was 10 seconds and the reaction time was 2 minutes. The activity unit (U) of cholinesterase was automatically printed out in the analyzer. The calculation of activity was performed according to the following formula:

$$U = \frac{\text{Decrease in absorbance at 340 nm}}{\text{Reaction time (5 min)}} \times$$

$$\frac{1}{\text{Molecular extinction coefficient } (\varepsilon} \times \frac{1}{\text{Light-path length (cm)}} \times \frac{1.005}{0.005} \times 10^3$$

$\varepsilon = 6.22 \times 10^3$ (Molecular extinction coefficient of NADPH)

The results of the measurements, repeated 30 times, were as follows:

Activity unit (U) = 180 ~ 350 (nmol/ml/min)

Coefficient of variation (CV) = 3.8%

Example 3

The procedure and calculation of Example 2 were repeated to determine activity unit (U) and Coefficient of variation (CV) but using 0.1 mM of m-hydroxybenzoyl choline chloride and 3 U of m-hydroxybenzoate hydroxylase instead of 0.1 mM of p-hydroxybenzoyl choline iodide and 0.6 U of p-hydroxybenzoate hydroxylase.

Activity unit (U) = 200 ~ 400 (nmol/ml/min)

Coefficient of variation (CV) = 3.79%

**Description for the Contracting States: CH DE FR GB LI NL**

This invention relates to a method for determining the activity of cholinesterase and to a diagnostic solution for use therein.

Two kinds of cholinesterase are known, these being generally referred to as true-cholinesterase and pseudocholinesterase. True-cholinesterase can be found in human red blood cells and nerve tissues. Pseudo-cholinesterase can be found in human sera and pancreas. Cholinesterase can decompose acylcholine (i.e. acetylcholine) to choline and acid (acetic acid).

When a person is subject to hepatic diseases or anaemia, the amount of cholinesterase in the blood decreases, and when a person is subject to nephrosis, diabetes mellitus or diseases of the nervous system, the amount of cholinesterase increases. A diagnosis of the diseases mentioned above can be made by measuring the level of cholinesterase in the blood. Therefore, the accurate determination of the activity of cholinesterase contained in a serum sample is significant from both the physiological and clinical viewpoints. The conventional methods for cholinesterase determination include (1) the Takahashi and Shibata method in which acetyl choline is used as a substrate and variation of the pH resulting from its decomposition is measured, and (2) a method in which benzoylcholine is used as a substrate, choline liberated from the benzoylcholine is oxidized with cholinoxidase to liberate hydrogen peroxide ($H_2O_2$), the hydrogen peroxide is subjected to reaction with phenol and 4-aminoantipyrine in the presence of peroxidase to produce a quinonimine dye, and the concentration of the quinonimine dye produced is colorimetrically measured.

However, such conventional methods have disadvantages. The above method (1) requires complicated steps and the above method (2) does not give very precise results.

One improved procedure for the determination of cholinesterase described in EP—A—0067 234 published as WO82/02212 on 8th July 1982 comprises use of p-hydroxybenzoylcholine derivatives of the general formula

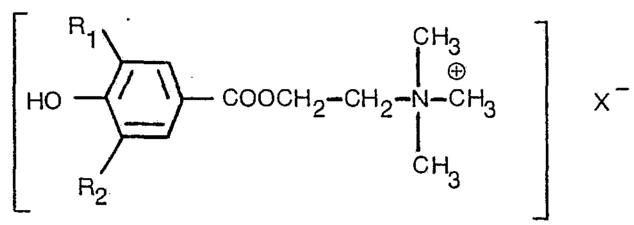

wherein $R_1$ and $R_2$ denote a hydrogen atom or one of the two a lower alkoxy group with 1 to 4 carbon atoms, X a halogen atom, as substrate, characterised in that p-hydroxybenzoic acid hydroxylase is reacted oxidatively in the presence of the co-enzyme nicotinamide-adenine-dinucleotide phosphate in reduced form with the p-hydroxybenzoic acid which is formed from the substrate under the action of cholinesterase, with the extinction loss difference or the acid usage in the reaction solution with the change of the nicotinamide-adenine-dinucleotide phosphate in the reduced form to oxidized form being read off or measured.

It is an object of the present invention to provide an alternative simple method for determining the activity of cholinesterase.

According to the present invention, there is provided a method of determining the activity of cholinesterase comprising the steps of:

mixing a liquid containing a cholinesterase with a substrate solution containing hydroxybenzoate hydroxylase, nicotinamide adenine dinucleotide phosphate in reduced form (NADPH) and m-hydroxybenzoyl choline having the following formula:

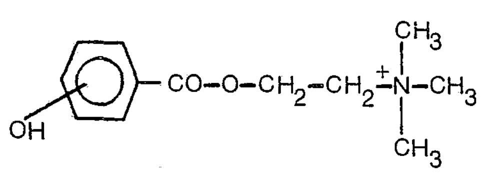

and

measuring the decrease in absorbance of light by NADPH as a measure of the activity of cholinesterase

in the solution thereby indicating the consumption of NADPH in accordance with the amount of cholinesterase present in said liquid.

A cholinesterase-containing liquid such as a serum is added to a solution containing m-hydroxybenzoate hydroxylase, nicotinamide adenine dinucleotide phosphate in reduced form (hereinafter referred to as NADPH) and m-hydroxybenzoyl choline. m-Hydroxybenzoic acid is produced from m-hydroxybenzoyl choline by the action of cholinesterase. The m-hydroxybenzoic acid is converted to dihydroxybenzoic acid by the action of hydroxybenzoate hydroxylase in the presence of NADPH. The consumption of NADPH is measured by measuring the decrease in absorbance of light by NADPH. The cholinesterase activity of the serum is determined from the decrease in absorbance by calculation.

The following examples illustrate the invention:

Example 1 (Preparative)

The synthetic substrate which is used in the method of the present invention, m-hydroxybenzoyl choline, was synthesized as follows:

A mixture of 5 g of m-hydroxybenzoic acid, 5 g of choline chloride and 5 ml of 18N $H_2SO_4$ was heated under reflux for 26 hours and then the pH of the reaction mixture was adjusted to 4.0.

The solid matter present was filtered off, and the filtrate was concentrated under reduced pressure. The concentrate was separated by silica gel chromatography to give m-hydroxybenzoyl choline chloride.

Example 2

To determine the activity of cholinesterase which is contained in a serum, 5 µl of the serum were added to 1 ml of a solution (pH 7.5) containing 0.1 mM of m-hydroxybenzoyl choline chloride, 3 U of m-hydroxybenzoate hydroxylase, 0.2 mM of NADPH and 20 mM of phosphate buffer. The decrease of the absorbance by the solution was measured at 340 nm of light at a temperature of 30°C during the course of the reaction. During the determination, use was made of a reaction rate analyser (LKB 2086 made and sold by Clinicon Co., Sweden) in which the time lag was 10 seconds and the reaction time was 2 minutes. The activity unit (U) of cholinesterase was automatically printed out in the analyzer. The calculation of activity was performed according to the following formula:

$$U = \frac{\text{Decrease in absorbance at 340 nm}}{\text{Reaction time (5 min)}} \times \frac{1}{\text{Molecular extinction coefficient } (\varepsilon)} \times \frac{1}{\text{Light-path length (cm)}} \times \frac{1.005}{0.005} \times 10^3$$

$\varepsilon = 6.22 \times 10^3$ (Molecular extinction coefficient of NADPH)

The results of the measurements, repeated 30 times, were as follows:

Activity unit (U) = 200 ~ 400 (nmol/ml/min)

Coefficient of variation (CV) = 3.79%

**Claims for the Contracting State: IT**

1. A method of determining the activity of cholinesterase comprising the steps of:
mixing a liquid containing a cholinesterase with a substrate solution containing hydroxybenzoate hydroxylase, nicotinamide adenine dinucleotide phosphate in reduced form (NADPH) and m- or p-hydroxybenzoyl choline having the following formula:

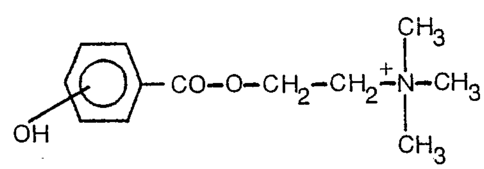

wherein the hydroxyl group is in the m- or p-position and measuring the decrease in absorbance of light by NADPH as a measure of the activity of cholinesterase in the solution thereby indicating the consumption of

NADPH in accordance with the amount of cholinesterase present in said liquid.

2. The method of claim 1, wherein said hydroxybenzoate hydroxylase is p-hydroxybenzoate hydroxylate and said hydroxybenzoyl choline is p-hydroxybenzoyl choline.

3. The method of claim 1 wherein said hydroxybenzoate hydroxylase is m-hydroxybenzoate hydroxylase and said hydroxybenzoyl choline is m-hydroxybenzoyl choline.

4. A diagnostic solution for use in determining the activity of cholinesterase, which comprises as active ingredients a hydroxybenzoate hydroxylase, nicotinamide adenine dinucleotide phosphate in reduced form and m- or p-hydroxybenzoyl choline.

**Claims for the Contracting States: CH DE FR GB LI NL**

1. A method of determining the activity of cholinesterase comprising the steps of:

mixing a liquid containing a cholinesterase with a substrate solution containing m-hydroxybenzoate hydroxylase, nicotinamide adenine dinucleotide phosphate in reduced form (NADPH) and m-hydroxybenzoyl choline having the following formula:

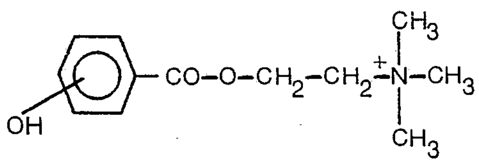

and measuring the decrease in absorbance of light by NADPH as a measure of the activity of cholinesterase in the solution thereby indicating the consumption of NADPH in accordance with the amount of cholinesterase present in said liquid.

2. A diagnostic solution for use in determining the activity of cholinesterase, which comprises as active ingredients m-hydroxybenzoate hydroxylase, nicotinamide adenine dinucleotide phosphate in reduced form and m-hydroxybenzoyl choline.

**Patentansprüche für den Vertragsstaat: IT**

1. Verfahren zur Bestimmung der Aktivität von Cholinesterase umfassend die Stufen:

Mischen einer eine Cholinesterase enthaltenden Flüssigkeit mit einer Grundlösung, welche Hydroxybenzoat-Hydroxylase, Nicotinamid- Adenin- Dinucleotid- Phosphat in reduzierter Form (NADPH) und m- oder p-Hydroxybenzoyl-Cholin mit der folgenden Formel

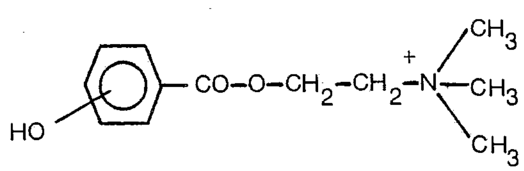

in der die Hydroxylgruppe in der m- oder p- Stellung ist, enthält, und

Bestimmen der Abnahme der Lichtabsorption durch NADPH als ein Maß für die Aktivität der Cholinesterase in der Lösung, und zwar auf Grund des Verbrauchs von NADPH entsprechend dem Anteil an Cholinesterase in der besagten Flüssigkeit.

2. Verfahren nach Anspruch 1, worin die besagte Hydroxybenzoat- Hydroxylase p-Hydroxybenzoat-Hydroxylase und das besagte Hydroxybenzoylcholin p-Hydroxybenzoylcholin sind.

3. Verfahren nach Anspruch 1, worin die besagte Hydroxybenzoat- Hydroxylase m-Hydroxybenzoat-Hydroxylase und das besagte Hydroxy-benzoylcholin m-Hydroxybenzoylcholin sind.

4. Diagnostische Lösung zur Verwendung bei der Bestimmung der Aktivität von Cholinesterase, welche als aktive Bestandteile eine Hydroxybenzoat- Hydroxylase, Nicotinamid- Adenin- Dinucleotid-Phosphat in reduzierter Form sowie m- oder p- Hydroxybenzoalcholin enthält.

**Patentansprüche für die Vertragsstaaten: CH DE FR GB LI NL**

1. Verfahren zur Bestimmung der Aktivität von Cholinesterasen umfassend die Stufen:

Vermischen einer Cholinesterase enthaltenden Flüssigkeit mit einer Grundlösung, welche m-Hydroxybenzoat-Hydroxylase, Nicotinamid-Adenin-Dinucleotid-Phosphat in reduzierter Form(NADPH) und m-Hydroxybenzoyl-Cholin mit der Formel

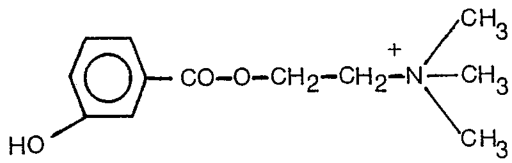

enthalt, und

Bestimmen der Abnahme der Lichtabsorption durch NADPH als ein Maß für die Aktivität der Cholinesterase in der Lösung, und zwar auf Grund des Verbrauchs von NADPH entsprechend dem Anteil an Cholinesterase in der besagten Flüssigkeit.

2. Diagnostische Lösung zur Verwendung bei der Bestimmung der Aktivität von Cholinesterase, welche als aktive Bestandteile m-Hydroxybenzoat-Hydroxylase, Nicotinamid-Adenin- Dinucleotid- Phosphat in reduzierter Form und m-Hydroxybenzoylcholin enthält.

**Revendications pour l'Etat contractant: IT**

1. Procédé de détermination de l'activité de la cholinestérase comprenant les stades consistant à mélanger un liquide contenant une cholinestérase avec une solution de substrat contenant l'hydroxybenzoate-hydroxylase, le phosphate de nicotinamide-adénine-dinucléotide sous forme réduite (NADPH) et la m- ou p-hydroxybenzoylcholine de formule suivante:

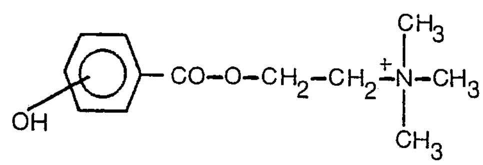

dans laquelle le groupe hydroxyle est en position *m* ou *p*, et à mesurer la diminution de l'absorbance de la lumière par le NADPH comme mesure de l'acitivité de la cholinestérase dans la solution indiquant ainsi la consommation de NADPH selon la quantité de cholinestérase présente dans ledit liquide.

2. Procédé selon la revendication 1, dans lequel l'hydrodroxybenzoate-hydroxylase est la p-hydroxybenzoate-hydroxylase et ladite hydroxybenzoylcholine est la p-hydroxybenzoylcholine.

3. Procédé selon la revendication 1, dans lequel ladite hydroxybenzoate-hydroxylase est la m-hydroxybenzoate-hydroxylase et ladite hydroxybenzoylcholine est la m-hydroxybenzoylcholine.

4. Solution diagnostique servant à déterminer l'activité de la cholinestérase, qui comprend à titre d'ingrédients actifs, une hydroxybenzoate-hydroxylase, le phosphate de nicotinamide-adénine-dinucléotide sous forme réduite et la m- ou p-hydroxybenzoylcholine.

**Revendications pour les Etats contractants: CH DE FR GB LI NL**

1. Procédé de détermination de l'activité de la cholinestérase comprenant les stades consistant à mélanger un liquide contenant une cholinestérase avec une solution de substrat contenant la m-hydroxybenzoate-hydroxylase, le phosphate de nicotinamide-adénine-dinucleóotide sous forme réduite (NADPH) et la m-hydroxybenzoylcholine de formule suivante:

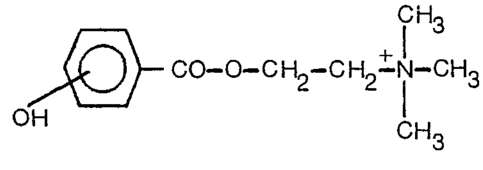

et à mesurer la diminution de l'absorbance de la lumière par le NADPH à titre de mesure de l'activité de la cholinestérase dans la solution indiquant ainsi la consommation de NADPH selon la quantité de cholinestérase présente dans ledit liquide.

2. Solution diagnostique servant à déterminer l'activité de la cholinestérase, qui comprend à titre d'ingrédients actifs la m-hydroxybenzoate-hydroxylase, le phosphate de nicotinamide-adénine-dinucléotide sous forme réduite et la m-hydroxybenzoylcholine.